# EUROPEAN PATENT APPLICATION

(11) **EP 2 682 118 A1**
(43) Date of publication of application: **08.01.2014**
(21) Application number: 12174673.9
(22) Date of filing: 02.07.2012
(51) Int. Cl.: A61K 31/35, A61P 9/00, A61P 9/04, A61P 9/12

(54) **Psoralen derivatives for the treatment of heart failure and heart hypertophy**

(71) Applicant: Max-Delbrück-Centrum für Molekulare Medizin Berlin-Buch, 13125 Berlin (DE)
(72) Inventor: Klussmann, Enno, 14163 Berlin (DE); Tröger, Jessica, 07580 Großenstein (DE); Rosenthal, Walter, 14153 Kleinmachnow (DE)
(74) Representative: Junghans, Claas

(57) **Abstract**

The present invention relates to compound characterized by a general formula 1, 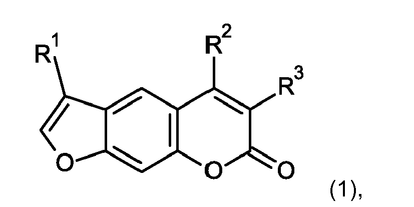 wherein
- R¹ is a aryl or a heteroaryl, and
- R² and R³
- independently of each other are hydrogen or a C₁-C₅ alkyl, or
- together are a C₃ or C₄ alkyl forming a 5- or 6 membered ring,

for use in a method for preventing or treating of heart failure, hypertension or cardiac hypertrophy.

## Description

The present invention relates to the use of psoralen derivatives in a method for preventing or treating heart failure, hypertension or cardiac hypertrophy, particularly caused by α- or β-adrenergic-receptor-induced hypertrophy of cardiomyocytes.

Protein kinase A (PKA) is a cAMP-dependent Ser/Thr kinase that plays a central role in cAMP-dependent signal transduction pathways. PKA consists of a dimer of regulatory (R) subunits, each of which binds one catalytic (C) subunit. The cAMP/PKA pathway controls a plethora of physiological processes.

AKAPs are a family of around 50 proteins. The defining characteristic of an AKAP is a PKA binding domain that interacts with the R subunit dimer of PKA. Some AKAPs interact with PKA RI type subunits, some with PKA RII type subunits, and some are dual specific interacting with both R subtypes.

The A kinase anchoring protein AKAP-Lbc tethers protein kinase A (PKA) and possesses a guanine nucleotide exchange factor (GEF) activity, which stimulates the small GTP-binding protein RhoA. In particular, AKAP-Lbc catalyzes the exchange of GDP to GTP in RhoA promoting its activation. RhoA controls a plethora of cellular processes including gene expression, cellular growth and cytoskeletal dynamics. Pathological changes of its function, in particular with regard to cytoskeletal structures, are often responses to chronic stress signals, a common characteristic of many diseases including chronic heart failure.

It has been shown that AKAP-Lbc is critical for activating RhoA and transducing hypertrophic signals downstream of 1-adrenergic receptors (ARs). Moreover, suppression of AKAP-Lbc expression by infecting rat neonatal ventricular cardiomyocytes with lentiviruses encoding AKAP-Lbc specific short hairpin RNAs strongly reduces both α1-AR-mediated RhoA activation and hypertrophic responses (Appert-Collin et al. PNAS, 104(24), 10140-10145, 2007).

Based on this background, it is the objective of the instant invention to provide novel inhibitors of the guanine nucleotide exchange factor activity of AKAP-Lbc for use in a method for preventing or treating heart failure, hypertension or cardiac hypertrophy.

It was surprisingly found that psoralen derivatives can specifically inhibit the guanine nucleotide exchange factor activity of AKAP-Lbc. Additionally, it has been found that by inhibition of the nucleotide exchange factor activity and the resulting inhibition of the RhoA activation, the psoralen derivatives of the present invention inhibit the α1-adrenergic induced increase in beating frequency of cardiomyocytes. The compounds provided by the present invention represent not only valuable tools, but also enable novel approaches for the treatment of diseases that involve AKAP-Lbc-mediated activation of RhoA such as chronic heart failure, hypertension or cardiac hypertrophy.

In general, the psoralen derivatives of the invention are characterized by having a mononuclear C₆ aryl or pentacyclic or hexacyclic heteroaryl in the 4' position of the psoralen scaffold (R¹), and optionally an alkyl substitution in position 3 or 4 (R³ and/or R²).

The term aryl in the context of the present invention signifies a cyclic aromatic hydrocarbon. A heteroaryl in the context of the present invention refers to an aryl that comprises one or several nitrogen, oxygen and/or sulphur atoms. The aryl substitution in position 4' may be substituted by one or more functional groups as defined below. Such functional group may enhance the solubility in an aqueous medium of the compound of the invention.

According to one aspect of the invention, a compound characterized by a general formula 1 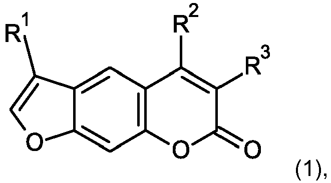 wherein
- R¹ is aryl or heteroaryl selected from the group comprised of: 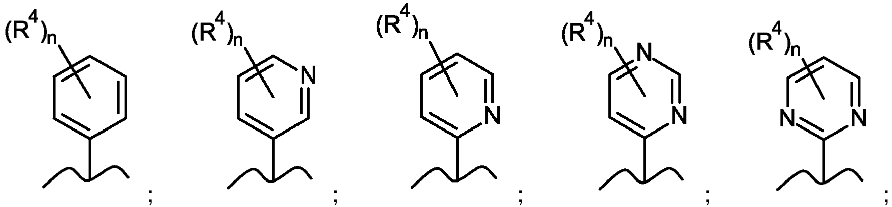 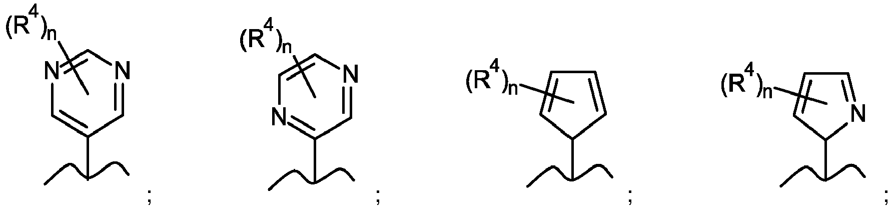 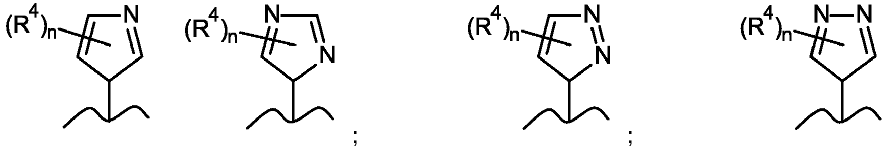 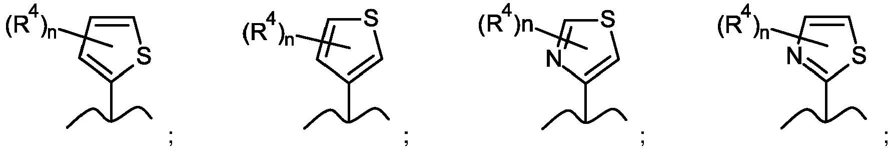 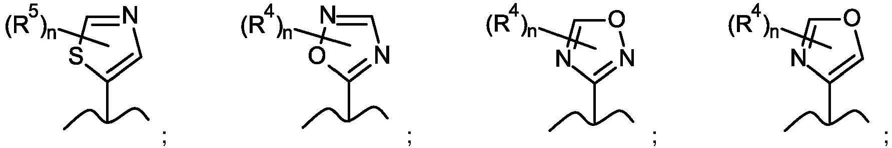 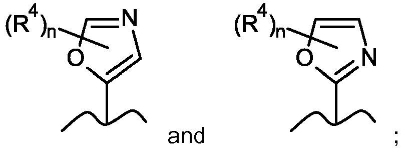 wherein
   n is 0, 1, 2, 3 or 4, and
   - each R⁴ independently from any other is COOR⁵ (carboxylic acid or ester), CONR⁵₂ (amide), C(NH)NR⁵₂ (substituted or unsubstituted amidine), CN₄H₂, NR⁵, COR⁵, OR⁵, CF₃, OCF₃, CN, NO₂, F, Cl or Br, or
   - two R⁴ together are a dioxyalkyl forming a five- or six-membered ring, and
- R² and R³
   - independently of each other are hydrogen or a C₁-C₅ alkyl, or
   - together are a C₃ or C₄ alkyl, forming a 5- or 6 membered ring,
      wherein each R² and R³ independently of the other bears 0, 1 or 2 substituents R⁶, with any non-substituted position being taken by hydrogen or fluorine, each R⁶ being selected, independently from any other, from COOR⁵, CONR⁵₂, C(NH)NR⁵₂, CN₄H₂, NR⁵₂, COR⁵, OR⁵, CF₃, OCF₃, CN, NO₂, Cl and Br,
   - with each R⁵ independently from any other being hydrogen or a C₁-C₄ alkyl,
is provided for the use in a method for preventing or treating cardiac hypertrophy, hypertension or heart failure.

A C₁-C₄ alkyl in the context of the present invention signifies a saturated linear or branched hydrocarbon having 1, 2, 3 or 4 carbon atoms, wherein one carbon-carbon bond may be unsaturated and one CH₂ moiety may be exchanged for oxygen (ether bridge). Non-limiting examples for a C₁-C₄ alkyl are methyl, ethyl, propyl, prop-2-enyl, n-butyl, 2-methylpropyl, tert-butyl, but-3-enyl, prop-2-inyl and but-3-inyl.

A C₁-C₅ alkyl in the context of the present invention signifies a saturated linear or branched hydrocarbon having 1, 2, 3, 4 or 5 carbon atoms, wherein one carbon-carbon bond may be unsaturated and one CH₂ moiety may be exchanged for oxygen (ether bridge). Non-limiting examples for a C₁-C₅ alkyl include the examples given for C₁-C₄ alkyl above, and additionally 3-methylbut-2-enyl, 2-methylbut-3-enyl, 3-methylbut-3-enyl, n-pentyl, 2-methylbutyl, 3-methylbutyl, 1,1-dimethylpropyl, 1,2-dimethylpropyl, 1,2-dimethylpropyl and pent-4-inyl.

In some embodiments, each R⁵ independently from any other is hydrogen, CH₃, C₂H₅, C₃H₇ or C₄H₉.

In some embodiments, R¹ is substituted by one or two R⁴ groups (n is 1 or 2).

In some embodiments, R¹ is phenyl, 2-pyridyl, 3-pyridyl, 4-pyridyl, 2-pyrimidyl, 4-pyrimidyl or 5-pyrimidyl. In some embodiments, R¹ is a six-membered ring substituted by one or two R⁴ substituents in para and/or ortho position to the attachment position of R⁴.

In some embodiments, R⁴ is a methoxy group (OCH₃). In some embodiments, R¹ is methoxyphenyl.

In some embodiments, R¹ is
- 2,3-dihydrobenzodioxine: 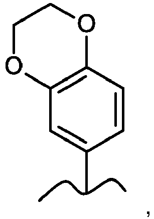 or
- 1,3-benzodioxole:

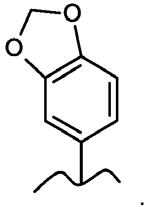

In some embodiments, one of R² and R³ is C₂H₅, C₃H₇, C₄H₉ or C₅H₁₁ and the other one is hydrogen or CH₃.

In some embodiments, one of R² and R³ is O(CHₓF₂₋ₓ)ₘCH_{y}F_{3-y} or (CH₂)ₘZ, wherein Z is selected from CH₃, (CH₂)ₓOR⁷, COOR⁷, CONR⁷,, with m being 0, 1, 2 or 3, x being 0, 1 or 2, y being 0, 1, 2 or 3, and R⁷ being hydrogen, CH₃ or C₂H₅.

In some embodiments, R² is methyl, ethyl, propyl, prop-2-enyl, n-butyl, 3-methylbut-2-enyl or 3-methylbut-3enyl.

In some embodiments, R³ is hydrogen or methyl.

In some embodiments, R² or R³ is substituted by one functional group selected from C(NH)NR⁵₂, CN₄H₂, NR⁵₂, COOR⁵, CONR⁵₂, COR⁵, CF₃, OCF₃, OR⁵, CN, NO₂, F, Cl, and Br, wherein each R⁵ independently from any other is hydrogen or a C₁-C₄ alkyl. In some embodiments, the functional group is in ω-position (terminal position on the alkyl chain).

In some embodiments, R² or R³ is substituted by a COOR", CONR"₂, CN₄H_{2,}, OR", OCF₃ or CF₃, wherein each R" independently from another is hydrogen, CH₃, C₂H₅, C₃H₇, or C₄H₉.

In some embodiments, R² is ω-hydroxy-n-propyl, n-propanol, ω-hydroxy-n-butyl or 2-hydroxy-2-methylpropyl.

In some embodiments, R² is a C₃ or C₄ alkyl and R³ is hydrogen.

In some embodiments, R² is a C₃ or C₄ alkyl and R³ is methyl. In some embodiments, R² is methyl and R³ is methyl.

According to some embodiments of the invention, a compound characterized by the general formula 1 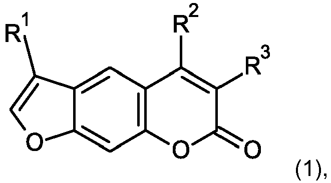 wherein
- R¹ is phenyl, 2-pyridyl, 3-pyridyl, 4-pyridyl, 2-pyrimidyl, 4-pyrimidyl or 5-pyrimidyl, and R¹ is substituted by n substituents, n being 0, 1 or 2, and each substituent independently from any other is OR⁷, COR⁷, COOR⁷, CONR⁷₂, CN, OCF₃, CF₃, F, Cl, Br, CN₄H₂, C(NH)NR⁷₂ or NO₂,
- R² is hydrogen, O(CHₓF₂₋ₓ)ₘCH_{y}F_{3-y} or (CH₂)ₘZ, wherein Z is selected from CH₃, (CH₂)ₓOR⁷, COOR⁷, CONR⁷,, with
   - m being 0, 1, 2 or 3,
   - x being 0, 1 or 2,
   - y being 0, 1, 2 or 3, and
   - R⁷ being hydrogen, CH₃ or C₂H₅,
- R³ is hydrogen, methyl, CH₂OH, ethyl, (CH₂)₂OH, propyl or (CH₂)₃OH, or
- R² and R³ together are a C₃ or C₄ alkyl forming a 5- or 6 membered ring,
is provided for use in a method for treating or preventing heart failure, hypertension or cardiac hypertrophy.

In one embodiment, the compound is selected from the group comprised of 3-(4-methoxyphenyl)-5-propyl-furo[3,2-g]chromen-7-one, 3-(4-methoxyphenyl)-5,6-dimethyl-furo[3,2-g]chromen-7-one and 5-butyl-3-(4-methoxyphenyl)furo[3,2-g]chromen-7-one.

The compounds shown in table 1 are preferred embodiments of any aspect of the invention.

**Table 1: Compounds of the invention:**

| Compound | Formula | Name |
|---|---|---|
| 31413 (IC50=25.9 µM) | 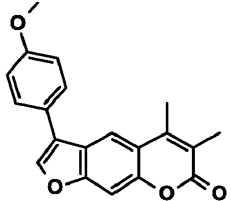 | 3-(4-methoxyphenyl)-5,6-dimethyl-furo[3,2-g]chromen-7-one |
| 31864 (IC50=39.9 µM) | 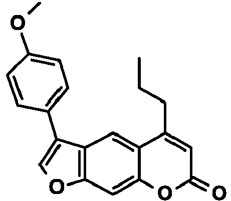 | 3-(4-methoxyphenyl)-5-propyl-furo[3,2-g]chromen-7-one |
| 31892 (IC50=78.9 µM) | 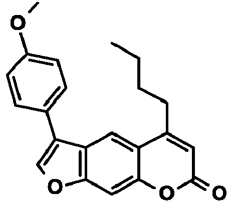 | 5-butyl-3-(4-methoxyphenyl)furo[3,2-g]chromen-7-one |

According to one aspect of the invention, a pharmaceutical composition for preventing or treating heart failure, hypertension or cardiac hypertrophy is provided, comprising a compound according to the above aspect or embodiments of the invention. Pharmaceutical compositions for enteral administration, such as nasal, buccal, rectal or, especially, oral administration, and for parenteral administration, such as subcutaneous, intravenous, intrahepatic or intramuscular administration, may be used. The pharmaceutical compositions comprise from approximately 1% to approximately 95% active ingredient, preferably from approximately 20% to approximately 90% active ingredient.

According to one aspect of the invention, a dosage form for preventing or treating heart failure, hypertension or cardiac hypertrophy is provided, comprising a compound according to the above aspect or embodiments of the invention. Dosage forms may be for enteral administration, such as nasal, buccal, rectal, transdermal or oral administration, or as an inhalation formulation or suppository. Alternatively, dosage forms may be for parenteral administration, such as intravenous, intrahepatic, or especially subcutaneous, or intramuscular injection forms. Optionally, a pharmaceutically acceptable carrier and/or excipient may be present.

According to one aspect of the invention, a method for manufacture of a medicament for preventing or treating heart failure, hypertension or cardiac hypertrophy is provided, comprising the use of a compound according to the above aspect or embodiments of the invention. Medicaments according to the invention are manufactured by methods known in the art, especially by conventional mixing, coating, granulating, dissolving or lyophilizing.

According to one aspect of the invention, a method for preventing or treating heart failure, hypertension or cardiac hypertrophy is provided, comprising the administration of a compound according to the above aspect or embodiments of the invention to a patient in need thereof.

The treatment may be for prophylactic or therapeutic purposes. For administration, a compound according to the above aspect of the invention is preferably provided in the form of a pharmaceutical preparation comprising the compound in chemically pure form and optionally a pharmaceutically acceptable carrier and optionally adjuvants. The compound is used in an amount effective against heart failure, hypertension or cardiac hypertrophy. The dosage of the compound depends upon the species, the patient age, weight, and individual condition, the individual pharmacokinetic data, mode of administration, and whether the administration is for prophylactic or therapeutic purposes. The daily dose administered ranges from approximately 1 µg/kg to approximately 1000 mg/kg, preferably from approximately 1 µg to approximately 100 µg, of the active agent according to the invention.

According to one aspect of the invention, a compound according to the above aspect or embodiments of the invention is provided for use in a method for preventing or treating cancer.

According to one aspect of the invention, the use of a compound as medicament is provided, wherein the compound is selected form the group comprised of 3-(4-methoxyphenyl)-5-propyl-furo[3,2-g]chromen-7-one, 3-(4-methoxyphenyl)-5,6-dimethyl-furo[3,2-g]chromen-7-one and 5-butyl-3-(4-methoxyphenyl)furo[3,2-g]chromen-7-one.

Wherever reference is made herein to an embodiment of the invention, and such embodiment only refers to one feature of the invention, it is intended that such embodiment may be combined with any other embodiment referring to a different feature. For example, every embodiment that defines R¹ may be combined with every embodiment that defines R² or R³, to characterize a group of compounds of the invention or a single compound of the invention with different properties.

The invention is further characterized, without limitations, by the following examples, from with further features, advantages or embodiments can be derived. The examples do not limit but illustrate the invention.

### Description of the figures

- Fig. 1: shows the concentration dependent inhibition of the nucleotide exchange by the compounds or the invention 31413, 31864 and 31892.
- Fig. 2: shows microscopic images and determined fluorescence intensities that illustrates the effect of compound 31413 on the formation of stress fibres.
- Fig. 3: shows the determination of the amount of active RhoA-GTP amount with SDS-PAGE.
- Fig. 4: shows the determination of the inhibitory effect of compound 31413 on different GEFs by measurement of luciferase activities.
- Fig. 5: shows the measurements of beat rate of neonatal cardiomyocytes in presence or absence of compound 31413.
- Fig. 6: shows the determination of the α-actin 1 mRNA amount in presence and absence of PE (phenylephrin) and compound 31413.
- Fig. 7: shows the determination of the α-actin 1 mRNA amount in presence and absence of PE, ISO (isoproterenol) and compound 31413.

### Examples

### Example 1: Inhibition of RhoA-GEF exchange

Following the protocol of the RhoGEF exchange assay Biochem Kit, a nucleotide exchange experiment for AKAP-Lbc and RhoA was established. The fluorescence labelled nucleotide analogues 2'(3')-O-(N-methylanthraniloyl)-guanosine diphosphate und 2'(3')-O-(N-methylanthraniloyl)-guanosine triphosphate, hereinafter referred to mGDP and mGTP, were used for tracing the nucleotide exchange.

### a) Nucleotide exchange of mGTP against GTPγS

25µl depletion buffer (100 mM sodium phosphate, pH 7.4, 5 mM EDTA, 200 mM ammonium sulfate) with an excess of mGDP (5 mM) were added to 200 µg recombinant his6-RhoA (25 µl) and incubated one hour at room temperature. Then, magnesium chloride (MgCl₂) was added to a final concentration of 10 mM.

By centrifugation with Vivaspin® 500 concentrator vials (MWCO 5 kDa, 10 min, 10000 rpm) and buffer (100 mM sodium phosphate, pH 7.4, 100 mM NaCl, protease inhibitor [complete, Mini, EDTA-free protease inhibitor cocktaile]) RhoA-mGDP was washed twice and reduced to 100µl. 1 µM RhoA-mGDP and 0,5 µM AKAP-Lbc DHPH in nucleotide exchange buffer (40 mM Tris pH 7.5; 100 mM NaCl; 20 mM MgCl₂, 100 µg/ml BSA) were used for the nucleotide exchange. The fluorescence measurements were performed in a micro plate reader (TECAN Safire) over a period of 15 min, whereby the fluorophor of mGDP was excited at 360 nm and the resulting fluorescence signal was determined at 440 nm.

### b) Nucleotide exchange of GDP against mGTP

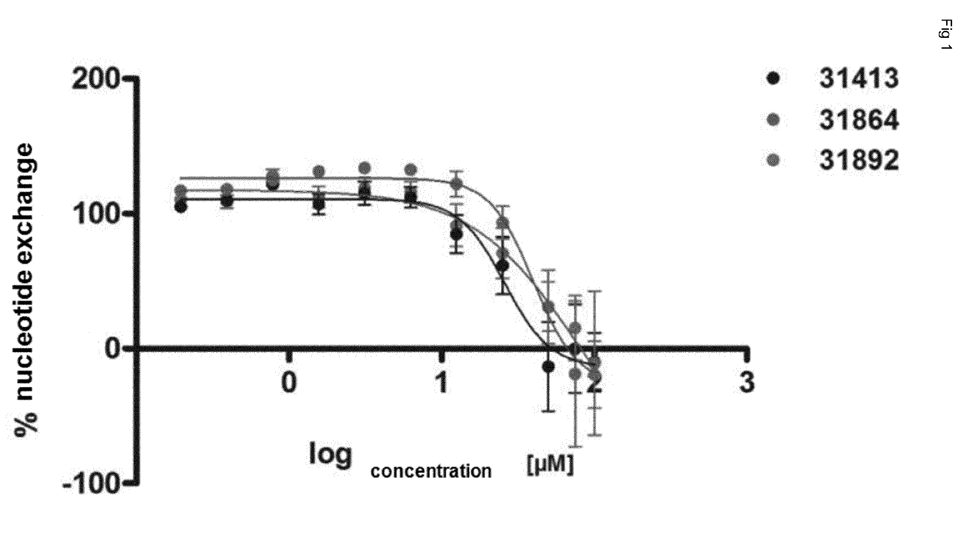

At a concentration relation of 2 µM protein and 1 µM mGTP, an ideal fluorescence signal-to-noise-ratio was found in the nucleotide exchange buffer (40 mM Tris pH 7.5, 100 mM NaCl, 20 mM MgCl₂, 100 µg/ml BSA). The fluorescence signal for mGTP was measured analogous to mGDP (λ_{exc}=360 nm; λₑₘ=440 nm) in a micro plate reader (TECAN Safire) over a period of 15 min. The experiment was performed in a 384-well plate. The compounds 31413, 31864 and 31892 (table 1) showed a fifty percent inhibition of nucleotide exchange in concentration between 25.9 µM and 78.9 µM (Fig. 1).

### Example 2: inhibition of stress fibre formation

For the initial analysis, 3T3cells were used to evaluate the cellular effects of the compounds of the present invention. In these cells changes in the cytoskeleton caused by GTPase activation are well observable, because the cells have a G protein coupled receptor for LPA. A stimulation of 3T3 cells with LPA leads to an activation of RhoA and thereby to the formation of fibrillar actin filaments (F-actin, stress fibres). The participation of AKAP-Lbc as an exchange factor for RhoA within the signal transduction via a G protein of the G_{α12/13} family was shown.

3T3 mouse fibroblasts were seeded on 30 mm cover slips (in 6 well plates) in a cell density of 2x10⁵ cells/well. After one day, the cells were cultivated without serum for 16 h to minimize the serum induced formation of stress fibres. On the following day, the cells were treated with 30 µM of compounds 31413, 31864 and 31892, respectively, for 30 min at 37 °C. After the treatment the formation of stress fibres were stimulated with 300 nM of lysophosphatide acid (LPA) for 10 min at 37 °C.

Following that, the cells were washed with phosphate buffer (Dulbecco's Phosphate-Buffered Saline ++) and fixed in 2.5 % paraformaldehyde in sodium cacodylate buffer (100 mM sodium cacodylate, 100 mM sucrose, pH 7.4) for 15 min. Then, the cells were permeabilized with 0.5 % Triton X-100. After incubation in a blocking solution (0.3 ml 45 % *fish skin* gelatine/100 ml PBS) for 30 min at 37 °C, the cells were treated with TRITC-Phalloidin. Phalloidin binds irreversibly to fibrillar actin (F-actin) and provides, coupled to the fluorescent dye tetramethyrhodamine isothiocyanate (TRITC), the visualization of the actin skeleton. Another dye, 4',6-diamidin-2-phenylindole (DAPI), binds to AT-rich region of the DNA and visualizes the nuclei. The stained specimens were bedded into a Immu-Mount 45 and stored in a dark, cooled place until the microscopic analysis. For the microscopic investigations, images were taken with two different fluorescence channels. The excitation was performed with argon-ion laser (364 nm) for DAPI and with a helium-neon-laser (543 nm) for TRITC-phalloidin.

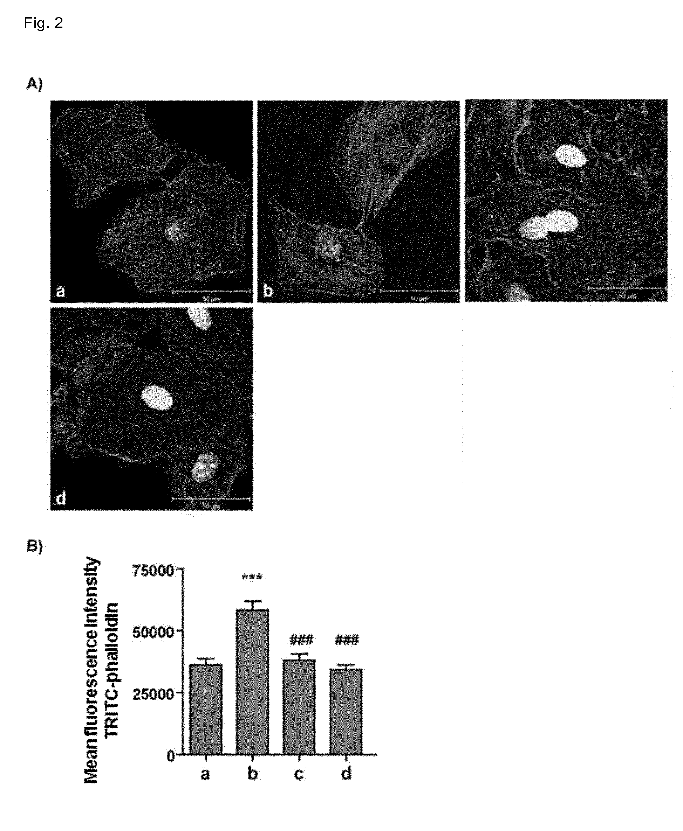

Without LPA stimulation no stress fibres could be identified after addition of DMSO instead of the compounds of the invention (Fig. 2A, a). After treatment with LPA a clear formation of stress fibres could be detected (Fig. 2A, b). The *Clostridium limosum* C3-ADP-ribosyltransferase (C3lim) was used as negative control, which inhibits RhoA by ADP-ribosylation at Asp 41. With C3lim the polymerization of actin and the formation of stress fibres were inhibited in the presence of LPA (Fig. 2A, c). Compound 31413 also showed a suppression of the stress fibre formation (Fig. 2A, d). The graph in Fig. 2B shows results of the immuno fluorescence measurement after quantification of the mean fluorescence intensity and supports the visual impression. All confocal images are representative examples of at least three independent experiments. The quantification of the stress fibre formation were performed with the software package ImageJ by the determination of the overall fluorescence of the TRITC-phalloidin. The statistical analysis was performed according to the method of analysis of variance (ANOVA). The significant difference to the untreated sample was ***, p < 0.001 and *, p < 0.05, respectively. The significance between the groups DMSO-LPA (Fig. 2B, b), C3slim+LPA (Fig. 2B, c) and 31413+LPA (Fig. 2B, d) was p<0,001, respectively.

### Example 3: Inhibition in vitro assays:

A luciferase reporter assay and a rhotekin precipitation assay were performed to investigate the RhoA activation in cells.

### a) Rhotekin-based precipitation

HEK293 cells were seeded in 6-well plates. One day later the cells were transfected with the following plasmid DNAs:

### Vector

| | |
|---|---|
| pEGFP-N1 | empty |
| pEGFP-N1 | human AKAP-Lbc (incl. N-term. Flag-tag) |
| pCMV2B | p63RhoGEF |
| pcDNA | GaqRC (constitutively active) |
| pcDNA | Ga12QL (constitutively active) |

After additional 24 hours the cells were treated with compound 31413 (50 µM) or DMSO for 30 min at 37 °C. Subsequently, the cells were lysed in precipitation buffer (50 mM Tris pH 7.4, 150 mM NaCl, 4 mM MgCl₂; 10 % v/v glycerol, 1 % octylphenoxypolyethoxyethanol [IPEGAL]) and centrifugated 20000 g for two minutes to remove cell debris. 40 µl of the lysate were taken for the determination of the total RhoA content. The residual lysate was incubated with 40 µl GSH-sepharose coupled GST-rhotekin for one hour at 4 °C. Then, the GSH-sepharose was washed twice with precipitation buffer, enriched with SDS-PAGE loading buffer and heated 5 min at 90 °C. The analysis of the precipitated RhoA-GTP was performed by SDS-PAGE and subsequent immunoblotting with anti-RhoA antibodies (sc-418). The ratio between total RhoA and Rho-GTP was densitometrically determined.

The activation state of RhoA was determined with a Rho binding region of the rhotekin protein, a Rho effector, immobilised on a sepharose bead in the presence and absence of compound 31413. Rhotekin only binds to the activated form of RhoA, RhoA-GTP. To investigate the effects of compound 31413 on G protein coupled receptor signals, HEK293 cells were transfected with a constitutively active form of the Gα12 subunit, with AKAP-Lbc (for the Gα12 mediated RhoA activation), with a constitutively active Gαq subunit (GαqRC) and with p63RhoGEF, respectively. p63RhoGEF contains the DNA sequence of a RhoA selective exchange factor and connects the Gαq coupled receptor signal with the activation of RhoA. The cells were treated with compound 31413 twenty four hours after transfection.

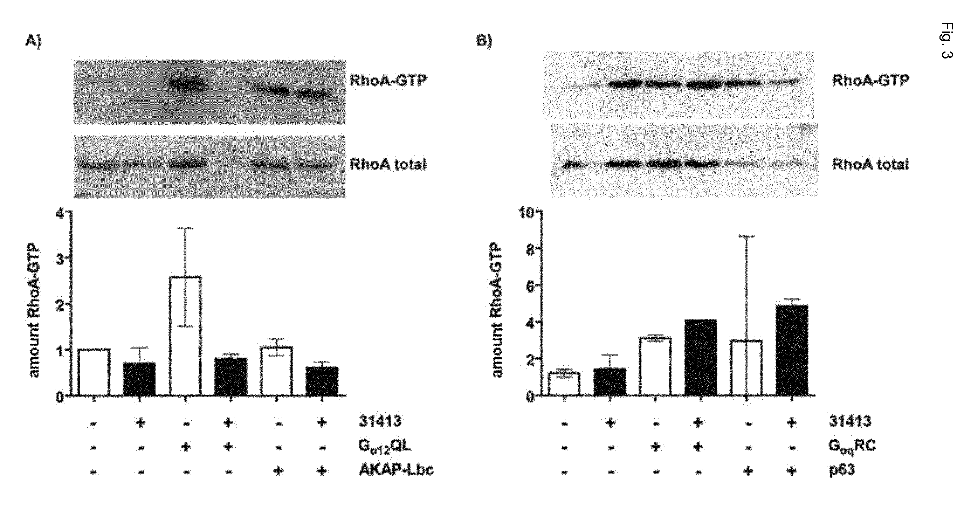

GTP-bound RhoA was detected by rhotekin precipitation (Fig.3). The precipitation of RhoA from transfected HEK293 cells was performed with a rhotekin sepharose, and analysed with SDS-PAGE and immunoblotting with a RhoA specific antibody. In contrast to the control cells treated with DMSO, the cells treated with compound 31413 shows a significantly decreased amount of activated GTP-bound RhoA (Fig.3A). The precipitated RhoA-GTP was separated with SDS-PAGE, blotted and detected with a specific anti-RhoA antibody.

For Gα12 coupled and AKAP-Lbc mediated activation of RhoA, less RhoA-GTP was precipitated in the presence of compound 31413 (Fig. 3, black bar) then in presence of DMSO (Fig. 3, white bar). For GαqRC and p63RhoGEF, no difference could be observed in the precipitated amount of RhoA-GTP of samples treated or untreated with compound 31413 (50 µM). This result indicates that compound 31413 influences the Gα12 coupled activation of RhoA and not the Gαq coupled activation.

### b) Luciferase-reporter assay

The experiments were performed following the protocol of the dual luciferase reporter assay (Promega). HEK293 cells were transfected with plasmid DNAs of two luciferases and plasmids of different exchange factors. A serum response element was located ahead of the coding luciferase sequences. Activated RhoA activates the serum response element, which enables the expression of the firefly luciferase. After 4 hours the cells were treated with compound 31413. After additional twenty four hours the cells were lysed in passive lysis buffer (Promega). The luciferase activity in the cell lysate was determined by measurement of the luminescence signal according to above mentioned protocol with 96 well plate (OptiPlate 96) and *Larll-* and. *Stop*-&*Glow*-substrate solutions in a micro plate reader

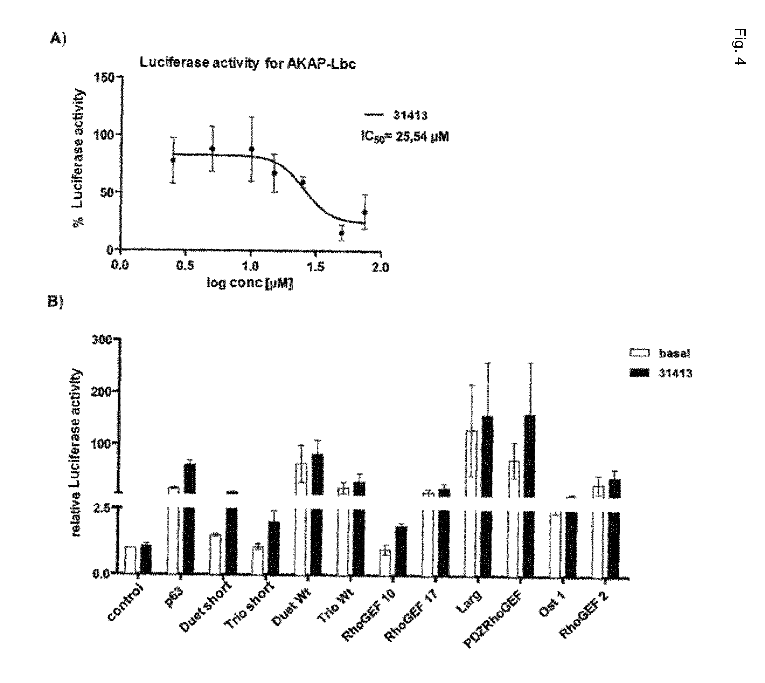

To rule out that other RhoGEF are influenced by the inventive compounds in their activity, HEK293 cells were transfected with different RhoGEF constructs. Additionally, as normalization control, two luciferase plasmids were added, which code for a serum response element (SRE) coupled firefly luciferase and for a *Renilla* luciferase, respectively. The GEF mediated activation of RhoA leads to an stimulation of the transcription factor serum response factor (SRF), which switches on the expression of the SRE-coupled luciferase. The cells were treated with compound 31413 four hours after transfection and lysed after twenty hours. The luciferase activities were determined in the lysate. First, the effect of compound 31413 on the AKAP-Lbc mediated RhoA/SRF activation was investigated (Fig. 4A). The luciferase activity was inhibited concentration dependently by compound 31413 with a determined IC₅₀ value of approx. 25 µM, which is comparable to the results from the nucleotide exchange experiment. Further, the luciferase activity of the indicated RhoGEFs was analysed in absence or presence of 50 µM compound 31413 (Fig. 4 B). None of the investigated GEF shows a significant reduction of the luciferase activity after treatment with compound 31413, when compared to the DMSO treated control. The data indicate the specific inhibition of the AKAP-Lbc mediated RhoA activation by the inventive compounds.

### Example 4: Effect of inventive compounds on cardiomyocytes

For cultivation of primary neonatal rat cardiomyocytes, hearts of two to three days old rats were obtained and quickly stored on ice in ADS buffer (116.4 mM NaCl, 5.4 mM KC, 5.6 mM dextrose,10.9 mM NaH₂PO4, 405.7 µM MgSO4, 20 mM Hepes, pH 7.3). Under sterile conditions, the hearts were cleaned from blood and vessels, cut into small pieces and transferred into falcon tubes. The ADS buffer was exchanged against an enzyme solution (see table below). Within four cycles each with five ml enzyme solution and an enzyme mix comprising collagenase and pancreatin, isolated cells were released from the tissue (12 min at 37 °C in a water bath with approx. 220 rpm).

| **Animal count** | **Collagenase** | **Pancreatin** | **in ADS-Puffer** | **FKS** | **Plat. Med.** |
|---|---|---|---|---|---|
| **Up to 20** | 12 mg | 14 mg | 18 ml (4 x 4 ml) | 2,5 ml | 5 ml |

The supernatant with the isolated cells was collected, transferred in fetal calf serum (FCS, 2.5 ml on ice) and subsequently centrifugated in FCS (100 g for 3 min at 4 °C). The supernatant of the centrifugation was discarded and the pellet was resuspended in 5 ml cold plating medium. After an additional centrifugation, the pellet was resuspended in 15 ml medium, transferred into a dish and incubated for two hours at 37 °C to allow the settling of the fibroblasts. The cardiomyocytes in the supernatant were counted with a Scepter Handheld Automated Cell Counter and transferred in dishes coated with gelatine. On the following day the medium was changed.

The neonatal cardiomyocytes were seeded in 6-well plates with 1,5x10⁶ cell per well. The stimulation with 100 µM phenylephrine (PE) was performed for six or twenty four hours at 37 °C. Untreated cells served as control. The effect of compound 31413 on the α-adrenergic stimulation of the cardiomyocytes was determined after incubation with the compound for three hours. To do this, the contraction of the cells was counted within a minute and different conditions were compared.

The frequency of contraction could be determined in a similar manner with a calcium sensor (Fluo-4). In a laser-scanning microscope, Fluo-4 was excited with an argon laser (488 nm) and the emission signals were detected and collected at a wavelength of 505 nm. The fluorescence intensity represented the intracellular concentration of calcium ions. Usually, the line-scan mode was used for image recording, and the line-scan images were recorded with a speed of 1.54 or 1.92 ms per line along the longitudinal axis of the cell. After the sequential image recording, a two-dimensional image with 512x100 lines or 512x2000 lines was generated and saved for later analysis.

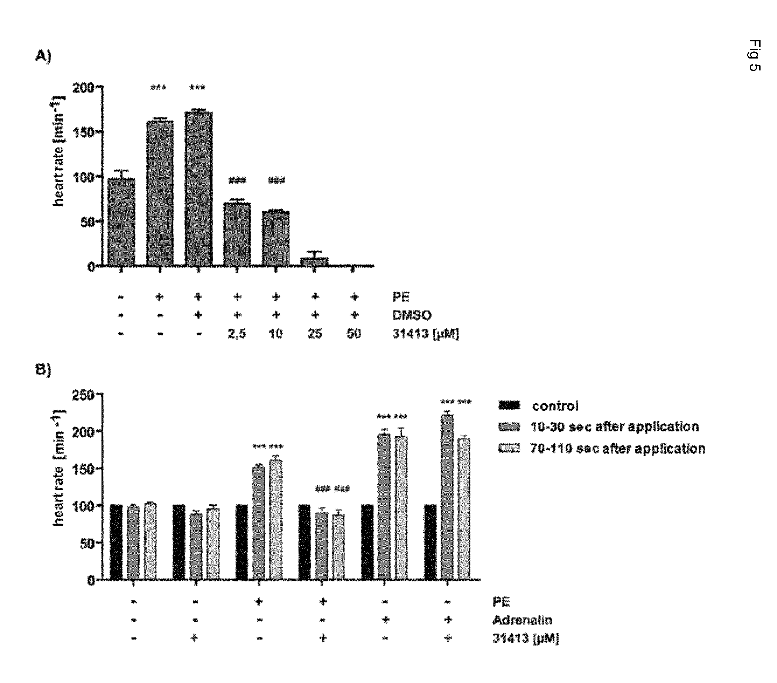

Isolated neonatal cardiomyocytes were used to investigate the relation between AKAP-Lbc induced RhoA activation and the contractility of the heart. After a chronic stimulation of the α1-adreno receptors (AR) with the specific agonist PE for six hours, the beats of the cardiomyocytes were counted over a period of 15 s and a beat rate per minute (bpm) was calculated. An increase of the beat rate of the cardiomyocytes from 100 bpm to 160 beats per minute could be observed. The solvent control (DMSO+PE) showed an equal result. The treatment of the cells with 2.5 µM compound 31413 led to a normalization of the beat rate. With significantly elevated concentrations of compound 31413 (25 µM and 50 µM), an arrest could be achieved (Fig. 5A). The beat rate of neonatal cardiomyocytes was counted with help of a light microscope.

Additional calcium measurements were performed. To do this, the cardiomyocytes were preincubated with 2.5 µM compound 31413. The fluorescence dye Fluo-4 was added after 20 min. Fluo-4 is a calcium sensor, which is used for investigating the calcium increase in cells. In cardiomyocytes the dye serves for examining the contractility. During contraction and relaxation, the calcium concentrations differ in the cytosol of cardiomyocytes. The fluorescence intensity of Fluo-4 increases with an increasing calcium concentration, and a decreasing calcium concentration will cause reduced fluorescence intensity. A frequency could be calculated from the fluo-4 fluorescence oscillation by a time depended analysis, wherein the fluorescence was measured over a period of two hours. The ground state at the moment before application (black), the period 10 to 30 sec after application (dark grey) and 70 to 110 sec after application (light grey) were evaluated (Fig. 5). Application refers to the adding of PE or adrenaline. The stimulation with PE and adrenaline leads to an increase of the frequency from 100 bpm to 150 bpm for PE and 200 bpm for adrenaline, respectively. The pre-incubation with 2.5 µM compound 31413 inhibited only the PE induced increase of beat rate and not the adrenaline induced one. This indicates an influence of compound 31413 on the α-AR-induced signals, because adrenaline mainly affects the β-adrenoreceptors. The results of the manual determination of the beat rate (Fig. 5A) are consistent with those of the calcium determination (Fig. 5B) and show a negative chronotropic effect of compound 31413 after α-adrenergic stimulation. The statistical analysis was performed according to the method of analysis of variance (ANOVA).. The significance between the groups PE and PE+31413 was p<0,001.

### Example 5: Inhibition of cardiac hypertrophy marker expression

Primary neonatal cardiomyocytes were prepared as described above. The cardiomyocytes were stimulated for 24 or 48 hours with 100µM phenylephrin (PE) or 10 µM isoproterenol (ISO) in absence or presence of 10µM or 50 µM of compound 31413.

After 24 hours or 48 hour of stimulation, the medium was removed, and every well was washed with 2ml phosphate buffered saline and fixed with 200 µl TRIzol. The cells were removed from each well and transferred into a 1.5 ml Eppendorf tube, respectively.

For cell breakdown, 40 µl chloroform was added to each Eppendorf tube. The broken cells were centrifuged for 15 min at 1400 g and 4 °C. The supernatant containing RNA/DNA was taken, mixed with 100 µl ispropanol and centrifugated for 20 min at 1500 g and 4 °C. The supernatant was discarded and the pellet was resuspended in 500 µL ethanol. The RNA from the pellet was purified by chromatography. The concentration of the RNA was determined by NANODROP.

The quantification of the hypertrophy marker α-sceletal muscle actin (α-actin 1) mRNA was performed by quantitative real-time PCR. First, the RNA was transcribed to a cDNA with a SuperScript^{™}III First-Strand Synthesis SuperMix (Invitrogen) and random hexamer primers. The cDNA was amplified in a PCR reaction in an Applied Biosystem StepOne^{™} Real-Time PCR System. Glycerinealdehydephosphate dehydrogenase and NTC (no template control) was used as controls. All samples were triplicates. The following primers have been used:

| Primer | Applied Biosystem catalogue number |
|---|---|
| ANF (Nppa) | 4351372, Rn00664637_g1 |
| α-sceletal muscle actin (ACTA1) | 4331182, Rn01426628_g1 |
| rat GAPD (GAPDH) endogenous Control | 4352338E |

| | |
|---|---|
| (VIC®/MGB Probe, Primer Limited) | |

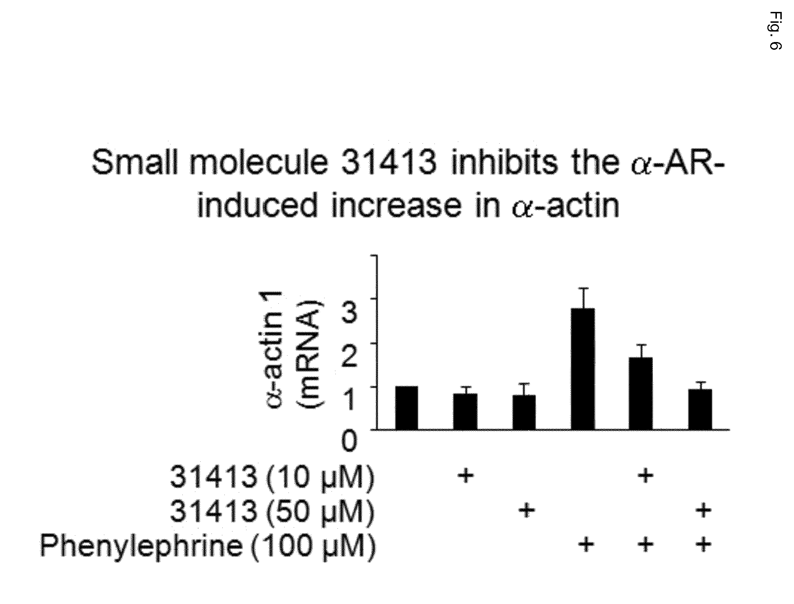

After 24 hours of stimulation wit PE (100 µM), a threefold increase in the relative amount of the hypertrophy marker α-sceletal muscle actin (α-actin 1) mRNA could be observed (Fig. 6). Without PE stimulation, the addition of compound 31413 caused no significant change in the relative amount of α-actin 1 mRNA. After PE stimulation and addition of compound 31413, a concentration dependent decrease of the α-actin 1 mRNA amount. This indicates the anti hypertrophic efficacy of compound 31413. The statistical analysis was performed according to the method of analysis of variance (ANOVA). The significance between the groups PE and PE+31413 was p<0,001.

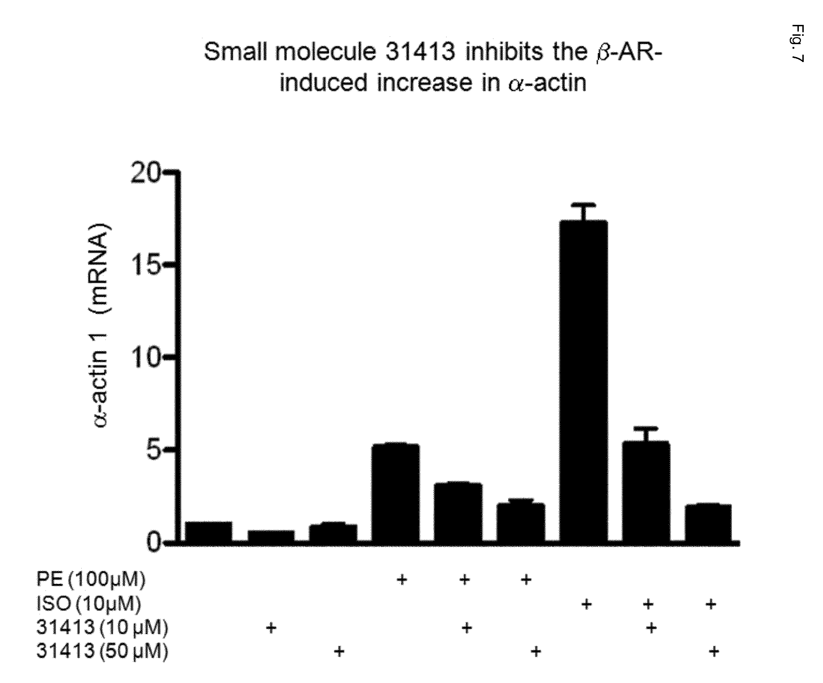

A significant decrease of the α-actin 1 mRNA amount could also be observed in the presence of 10 µM and 50 µM compound after stimulation with ISO for 48 hours (Fig. 7). This indicates that b-adrenergic induced hypertrophy can also be inhibited by the compounds of the invention.

## Claims

1. A compound **characterized by** a general formula 1 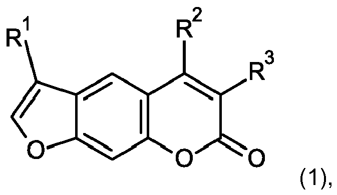 wherein
- R¹ is an aryl or a heteroaryl selected from the group comprised of: 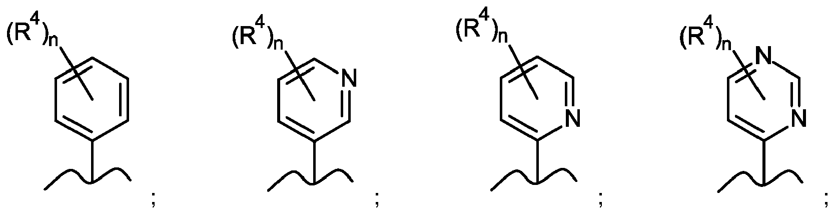 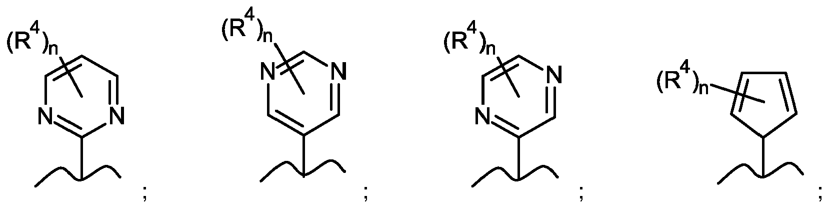 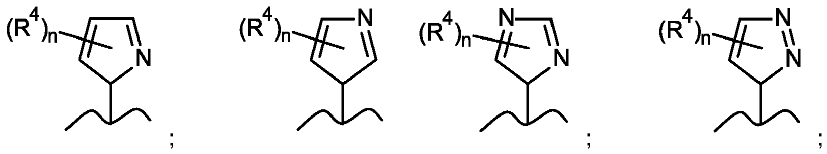 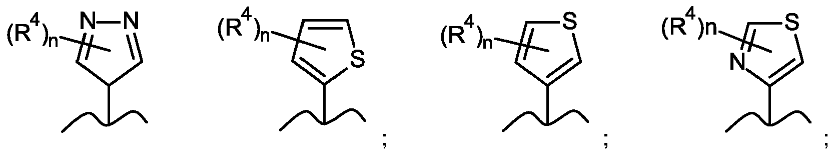 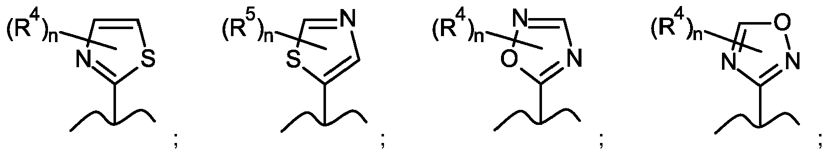 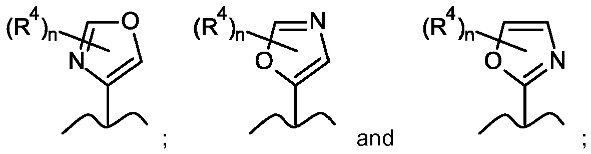 wherein
n is 0, 1, 2, 3 or 4, and
- each R⁴ independently from any other is COOR⁵, CONR⁵₂, C(NH)NR⁵₂, CN₄H₂, NR⁵₂, COR⁵, OR⁵, CF₃, OCF₃, CN, NO₂, F, Cl or Br, or
- two R⁴ together are a dioxylalkyl forming a five- or six-membered ring, and
- R² and R³
- independently of each other are hydrogen or a C₁-C₅ alkyl, or
- together are a C₃ or C₄ alkyl forming a 5- or 6 membered ring,
wherein each R² and R³ independently of the other bears 0, 1 or 2 substituents R⁶, with any non-substituted position being hydrogen or fluorine, each R⁶ being selected, independently from any other, from COOR⁵, CONR⁵₂, C(NH)NR⁵₂, CN₄H₂, NR⁵₂, COR⁵, OR⁵, CF₃, OCF₃, CN, NO₂, Cl and Br,
- with each R⁵ independently from any other being hydrogen or a C₁-C₄ alkyl, for use in a method for preventing or treating heart failure, hypertension or cardiac hypertrophy.

2. A compound **characterized by** the general formula 1, 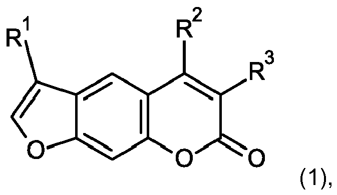 wherein
- R¹ is phenyl, 2-pyridyl, 3-pyridyl, 4-pyridyl, 2-pyrimidyl, 4-pyrimidyl or 5-pyrimidyl, and R¹ is substituted by n substituents, n being 0, 1 or 2, and each substituent independently from any other is OR⁷, COR⁷, COOR⁷, CONR⁷₂, CN, OCF₃, CF₃, F, Cl, Br, CN₄H₂, C(NH)NR⁷₂ or NO₂,
- R² is hydrogen, O(CHₓF₂₋ₓ)ₘCH_{y}F_{3-y} or (CH₂)ₘZ, wherein Z is selected from CH₃, (CH₂)ₓOR⁷, COOR⁷, CONR⁷, with
- m being 0, 1, 2 or 3,
- x being 0, 1 or 2,
- y being 0, 1, 2 or 3, and
- R⁷ being hydrogen, CH₃ or C₂H₅,
- R³ is hydrogen, methyl, CH₂OH, ethyl, (CH₂)₂OH, propyl or (CH₂)₃OH, or
- R² and R³ together are a C₃ or C₄ alkyl forming a 5- or 6 membered ring,
for use in a method for preventing or treating heart failure, hypertension or cardiac hypertrophy.

3. A compound according to claim 1 or 2, said compound selected form the group comprised of:
- 3-(4-methoxyphenyl)-5-propyl-furo[3,2-g]chromen-7-one;
- 3-(4-methoxyphenyl)-5,6-dimethyl-furo[3,2-g]chromen-7-one;
- 5-butyl-3-(4-methoxyphenyl)furo[3,2-g]chromen-7-one.

4. A pharmaceutical composition for preventing or treating heart failure, hypertension of cardiac hypertrophy, comprising a compound according to any of claims 1 to 3.

5. A dosage form for preventing or treating heart failure, hypertension or cardiac hypertrophy, comprising a compound according to any of claims 1 to 3.

6. A method for making a medicament for preventing or treating heart failure, hypertension or cardiac hypertrophy, comprising the use of a compound according to any of claims 1 to 3.

7. A method for treating heart failure or cardiac hypertrophy, comprising the administration of a compound according to any of claims 1 to 3 to a patient in need thereof.

8. A compound according to any of claims 1 to 3, for use in a method for preventing or treating cancer.

9. Use of a compound as a medicament, said compound selected form the group comprised of:
- 3-(4-methoxyphenyl)-5-propyl-furo[3,2-g]chromen-7-one;
- 3-(4-methoxyphenyl)-5,6-dimethyl-furo[3,2-g]chromen-7-one;
- 5-butyl-3-(4-methoxyphenyl)furo[3,2-g]chromen-7-one.
